Europäisches Patentamt

European Patent Office

Office européen des brevets

(11) Publication number: **0 503 182 A1**

# EUROPEAN PATENT APPLICATION

(21) Application number: **91304435.0**

(22) Date of filing: **17.05.91**

(51) Int. Cl.5: **A63B 23/16**

(30) Priority: **08.03.91 US 666229**

(43) Date of publication of application:
**16.09.92 Bulletin 92/38**

(84) Designated Contracting States:
**DE FR GB IT**

(71) Applicant: **Donohue, Patrick T.**
**1822 N.E. 143rd,**
**Portland, Oregon 97230(US)**

(72) Inventor: **Donohue, Patrick T.**
**1822 N.E. 143rd,**
**Portland, Oregon 97230(US)**

(74) Representative: **Marlow, Nicholas Simon et al**
**Reddie & Grose 16, Theobalds Road**
**London WC1X 8PL(GB)**

(54) **Modular digital traction system.**

(57) A therapeutic traction system 10 comprises two operational modules, a bandage-like traction band module 26,28 for applying to a finger or other digit 12,14 and a spring module 30. A working combination usually consists of two traction band modules with a spring module 30 connected between them so as to bias them apart. Traction band modules 26,28 may vary in the number and orientation of connecting elements 46,48 they carry and to which a spring module 30 may be connected. Spring modules are elongated coil springs which, in use, are usually deflected into a bow form and, optionally, also torsionally deflected, to create the desired biasing force. A third auxiliary module of the system is a tool 32 for holding the springs in a desired bowed configuration and, optionally, with some torsional preload, to facilitate mounting a given combination of traction bands 26,28 and spring module 30 onto the digit 12,14.

FIG. 1

## BACKGROUND OF THE INVENTION

The invention concerns orthotic devices for applying forces or pressures at the joints of the human body and more particularly for applying traction and other manipulative forces at the digits of the extremities, especially the hands.

For convenience, discussion and description will be limited largely to embodiments of the invention applied to the hand. But it will be clear that the principle of the invention may have application at the joints of other body members and particularly the feet.

Certain non-malignant conditions, such as arthritis, repetitive motion injury, trauma and the like, cause unnatural pull on the muscles, tendons, and ligaments of the hand resulting in painful joints, deformities and sometimes partial or total loss of use of the hand. Pain and loss of joint strength and range of motion may be experienced because a phalanx drifts or is pulled out of its normal position in relation to another phalanx or the metacarpal bone, causing the respective joint to become deformed. Or the tissues or phalanges of a digit may degenerate as a result of disease and the like, resulting in deformities (misalignment) or distortions of the digit.

Devices which apply an axial force (traction) across a joint, or opposing lateral forces on opposite sides of a joint, tend to:

1. Relieve pain by lowering the pressure within the joint.
2. Strengthen the muscles, tendons, and ligaments.
3. Restore the joint's normal range of motion.
4. Act as an opposing force to undesirable joint contractive forces which can cause deformities.
5. Facilitate the healing process.

A number of devices of this general type are known but while they may function satisfactorily they suffer from a variety of limitations. Most of them are single purpose, providing only one function, and individual units may require custom fitting to particular digits or to the extremity as a whole and may not be readily adaptable to another application. Some devices severely limit the mobility of the individual digits or of the whole extremity. The net cost of using such devices may be relatively high, both because of the high cost of manufacture of a relatively complicated design and the duplication required because of lack of adaptability of the design. For example, the orthotic devices for use on the extremities disclosed in U.S. patents 3,533,405 Collins, 3,595,225 Beeman and 4,220,334 Kanamoto all have a single purpose or mode of application. No device is known which provides two or more functions from a group of functions comprising: applying traction; applying opposing lateral forces at a joint; and applying separating forces between two digits. No simple device is known for applying traction at an individual interphalangeal joint of a digit.

## SUMMARY OF THE INVENTION

Accordingly preferred embodiments of the invention provide a simple low cost orthotic arrangement for influencing the joints of the body and particularly interphalangeal and other joints of the extremities. Preferably the arrangement has more than one mode of use, both in terms of the nature of the forces applied and the positions on the extremity at which they are applied.

In one form the invention may comprise a band for mounting on a digit of an extremity and facilitating the application of a manipulative force to the digit, the band being engageable with the digit so as to substantially encircle it and so grip the digit that the band is displaced neither axially nor rotationally when the band is subjected to an externally applied manipulative force, the application of the band to the digit establishing a generally cylindrical external surface of the band, the cylindrical surface having a longitudinal axis coinciding approximately with the longitudinal axis of the digit, and the band being characterized in that the cylindrical surface carries at least one connecting element, said element being shaped so as to define at least one directional axis and to be connectible with an elongated resilient force exerting member, said member having opposite ends and each end having a longitudinal axis, and so that, when connected, the at least one directional axis and the longitudinal axis of the force exerting member end to which it is connected are parallel.

The invention may also comprise a system for applying therapeutic forces to an elongated body member at a joint of the member, the member having a longitudinal axis and the system including a pair of longitudinally spaced anchoring means including a first anchoring means comprising a band for substantially encircling and gripping a first portion of the body member, and elongated resilient biasing means extending between the members of the pair of anchoring means, the system being characterized in that the elongated resilient biasing means is connected and configured so as to bias apart the members of the pair of anchoring means in a direction generally parallel to the longitudinal axis of the body member.

Preferred embodiments of the invention provide maximum versatility of application with a minimum number of configurations or variations of individual component of the systems.

Preferred embodiments of the invention also provide a traction system which may function to apply traction, to apply opposing lateral forces at a joint, or to apply separating forces between two digits. Another object is to provide an arrangement for applying traction at an individual interphalangeal joint.

The system may comprise two basic operational modules and one auxiliary module or tool. The operational modules may comprise a traction band, for substantially encircling a digit and gripping it so that the band is not displaced when an external therapeutic force is applied to the band and hence into the digit, and an elongated resilient biasing element. The auxiliary element may comprise a tool for holding a given combination of operational modules in a selected configuration or at a selected level of preload while the device is being mounted onto the digit.

Each traction band carries at least one connecting element accessible externally of the band when the band is in position on a digit. Opposite ends of the resilient biasing element are each connectable to a connecting element of a traction band. Generally a minimum functioning unit or combination of the modular traction system consists of two traction bands mounted on an extremity with, for example, one band on each side of an interphalangeal joint or one band on each of adjacent digits, with a single resilient biasing element connected between them in such a way as to bias them apart in a direction generally parallel to the plane containing the longitudinal axes of the digits. An advantage of the system is that the biasing element may "bridge" the portion of the extremity being treated (one or two interphalangeal joints for example) so that there is no direct contact with the treated portion and it may remain exposed.

Another preferred embodiment of the invention provides a traction band module for a digital traction system which is readily and reliably secured to a digit, and which incorporates a connecting element for connection to an external biasing element so that an external force may be applied to the digit. Preferably the traction band is adaptable to a range of digit sizes and its structure facilitates low cost manufacture so that it may be considered a disposable or throwaway module of the traction system.

Preferred embodiments of the invention may include a traction band in the form of a flexible bandage for wrapping a digit and having a soft inner lining of foam rubber or the like with an adhesive coating to secure the band and prevent it from slipping on the digit. The flexible but stiffer outer layer of the band may carry the connecting element either formed as an integral part of the outer layer or cover or at least rigidly attached to it.

The resilient biasing element may be an elongated spring having opposite ends, each end being releasably connectable with a connecting element of a traction band.

Preferably each connecting element is formed so that it has at least one and preferably two directional axes and so that when a spring is connected the longitudinal axis of the end of the spring extends in a fixed predetermined and selected direction, with respect to the traction band and parallel to one of the directional axes. Advantageously, a modular traction system according to the invention may include interchangeable biasing element modules (springs) of various strengths, to suit particular applications.

Traction bands may carry more than one connecting element so that three or more traction bands may be used in combination (connected by two or more biasing elements). Orientation of the directional axes of the connecting elements on any one traction band may be mixed, so that in an application of three or more traction bands to an extremity more than one spring configuration and force mode may be used.

Preferably the spring is an elongated coil spring wound with initial tension so that it stores potential energy efficiently when subjected to either bending or torsional deflection. The force applied at a connecting element of a traction band may thus be a simple translational force or a combination of translational force and torsion.

In use the elongated resilient element may be a coil spring formed into a bow or arch when the device is mounted onto the fingers. An auxiliary member (tool) in the form of a restraining band may be used to hold the spring in the desired bowed configuration while the associated two traction bands are being applied to the finger. In applications where the spring is torsionally deflected before mounting so as to store torsional energy and hence produce lateral forces at the joint being treated, the same tool may be used as a wrench to hold the spring in its pre-wound position while it is being mounted onto the patient's hand.

Preferred embodiments of the invention provide a traction system which may be used to load directly a metacarpophalangeal (mcp) joint by mounting a traction band on a proximal phalange adjacent the mcp joint.

A traction system according to the invention is thus versatile, simple and potentially low in cost. Its simplicity and lower cost particularly qualify it for self-application and management by the patient with a minimum of physician supervision.

## BRIEF DESCRIPTION OF THE DRAWINGS

Fig. 1 is a semi-schematic perspective view of modules of a traction system according to the invention assembled into a possible arrangement for two adjacent fingers and including two different embodiments of a traction band and showing a representative auxiliary element (tool).

Fig. 2 is an enlarged cross-sectional view taken on line 2-2 of Fig. 1 showing a traction band and the engagement of a spring module with the connecting element of the traction band.

Fig. 3 is also an enlarged view, in perspective, showing a single connecting element component of a traction band.

Fig. 4 shows a traction system assembly including two traction bands ready for installation on a digit with a spring bowed between them and held in that configuration by a restraining tool.

Fig. 5 is a view similar to Fig. 4 showing the restrainer tool in its wrench mode, in position to receive and engage a connecting element which has already been torsionally deflected.

DESCRIPTION OF THE PREFERRED EMBODIMENTS

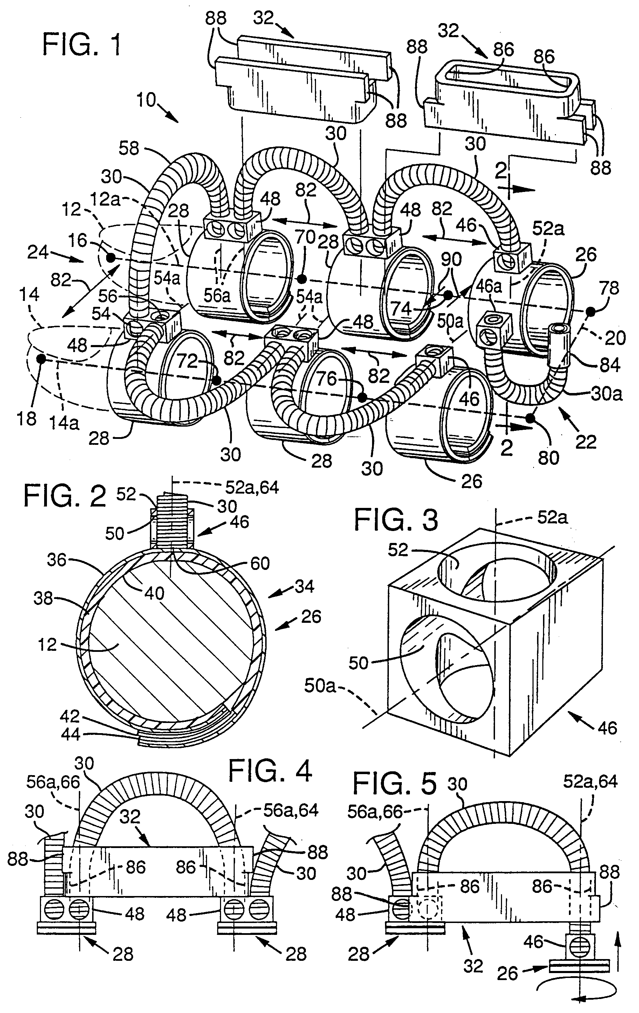

The invention is embodied in the exemplary traction system arrangement 10 shown in Fig. 1. The system is usable to develop manipulative forces at the joints other than pure traction but for convenience it will be referred to as a traction system. The system concepts may be embodied in devices adapted for use at the joints of fingers and toes and at other joints such as wrists, knees and elbows, but, again for convenience, the following description and discussion will usually refer only to the digits of the hand.

The arrangement 10 of Fig. 1 is shown semi-schematically. Although such an arrangement of traction system modules may feasibly be applied to a hand in practice, the main purpose of the figure is to show, in a single grouping, two exemplary embodiments of traction band module according to the invention and some examples of how they may be used together, in conjunction with a spring module. The system 10 is mounted on adjacent first and second digits 12 and 14 respectively indicated mainly by their longitudinal axes 12a, 14a. Each finger has a tip 16, 18 respectively. Finger crotch 20 extends between the fingers. The traction system arrangement 10 thus has a palm end 22, and a finger tip end 24.

The traction system according to the invention is made up of traction band, spring and tool modules. In Fig. 1, first and second embodiments of traction band 26, 28 are shown along with single embodiments of spring module 30 and of tool module 32.

Common elements of all traction band modules are a band portion 34 for substantially wrapping and securing the traction band to a digit and a connecting element (46,48) for connecting it to a second module such as spring 30. The common features of the structure of traction band are seen best in the cross-sectional view in Fig. 2 of the first traction band 26. The layered construction of the band portion 34, from outside to inside, consists of: thin plastic sheet material forming an outer cover 36, generally cylindrical when in use; a somewhat resilient lining 38 which may be of foam rubber; and, preferably, an adhesive coated inner face 40 of the lining 38. A peel-off backing strip (not shown in the drawings) may be used, conventionally, to protect the adhesive face 40 until the traction band is about to be mounted onto the digit. Mating patches of Velcro (TM) type hook and loop fastener material 42, 44, respectively are mounted on the opposite ends and on opposite sides of the cover strip 36 to help secure the band to the digit. The (supplementary) Velcro (TM) type fastener is not essential and, in some cases, a patient may find its absence simplifies mounting of the device.

Each traction band 26, 28 carries at least one connecting element 46, 48 and these two embodiments of traction band differ only in having either a single connecting element 46 (first traction band 26) or a dual connecting element 48 (second traction band 28). The single connecting element 46 has a simple cubical form with mutually perpendicular through holes 50, 52 defining cylindrical apertures or receptacles with longitudinal axes 50a, 52a, respectively which may be regarded as directional axes as explained below. The dual connecting element 48 of the second traction band 28 may simply be the equivalent of two single connecting elements 46 side-by-side and integrated into one unit. The dual element has pairs of mutually perpendicular through holes 54, 56 with respective longitudinal axes 54a, 56a which axes may also be regarded as directional axes as explained below.

The connecting elements 46, 48 may be of a suitable plastic material and, either attached to the cover strip 36 by a suitable method such as cementing or hot fusion, or may be formed integrally with the cover strip 36 by a suitable method such as injection molding.

Spring 30 of the spring module is preferably a coil spring of steel wire closely wound, with initial tension between the coils 58, and preferably with squared ends - see end 60 in Fig. 2. First and second end portions of the longitudinal axis of the spring are designated 64, 66, respectively as indicated in Fig. 5. Other forms of elongated spring or spring material having suitable beam deflection (and preferably torsional deflection) characteristics may be used. For example, solid spring steel wire may be used, possibly partially bowed in its free state (not shown).

An important aspect of a traction system according to the invention is the connection of the spring module 30 to the traction band 26, 28. Fig. 2 illustrates the seating of a spring module end 60 in the hole or receptacle 52 of a connecting element 46. Preferably the spring end 60 is held firmly but releasably by the connecting element 46 so that their respective longitudinal axes 52a, 64 approximately coincide (or at least are approximately parallel). Preferably the grip of the spring by the connecting element is sufficient to allow some torsional deflection to be applied to the spring before mounting the band onto the digit and maintained while in use. The tightly wound coil spring of this exemplary embodiment is particularly advantageous in this application in that, although the fit of the spring in the connecting element after insertion may be adequately tight, the well known, slight diameter reducing yielding of the spring under torsion (in one direction) allows it to be readily twisted into and out of engagement when turned in the appropriate direction. Also, the fit of the spring end 60 in the connecting element 46 may be such that the spring may be "snapped" into and out of engagement. A particular "male/female" connection relationship is illustrated here. Clearly the relationship could be reversed with the connecting element 46 becoming a pin, entering the end 60 of the spring (an arrangement not shown in the drawings). Preferably, in any alternative connection arrangement, the connecting element carried by the traction band should define a directional axis determining the alignment of the spring at the point of connection. Clearly, the modular traction system may include interchangeable springs (biasing elements) 30 of various strengths and perhaps lengths to suit particular applications. To achieve this, wire size or initial tension, for example, may be changed.

Fig. 2 also illustrates the traction band 26 firmly gripping the finger 12. The resilient liner 38 both increases comfort for the patient and helps to stabilize the traction band on the finger. The use of the mating Velcro (TM) elements 42, 44 enhances the security of the band on the finger and also facilitates limited reusability of the band. Preferably the traction band cover portion 36 is sufficiently stiff that, in assembly, it defines a relatively regular cylindrical form and provides a substantially non-yielding base for the connecting element 46 so that any force applied to the connecting element is transferred effectively and efficiently to the finger 12. In keeping with the invention, other forms of traction band construction may be used. But it is important that a band provide support for a connecting element, such as elements 46, 48, which is stable and secure so that an external therapeutic force may be transferred comfortably and efficiently to the digit on which it is mounted. In particular, any band securing method must hold the band against axial or rotational movement on the digit. For hygienic and other reasons a preferred construction of traction band is one which makes the band affordably disposable, similar for example to the construction shown most clearly in Fig. 2.

An indication of the versatility of a traction system according to the invention is given by the "demonstration" arrangement 10 of Fig. 1 which produces both traction at individual interphalangeal joints and a separation force between the digits. The traction bands 26, 28 are each of the same general construction (seen best in Fig. 2) and are mounted on the respective distal, middle and proximal phalanges of the fingers 12, 14 so that there is a traction band on each side of the distal and proximal interphalangeal joints 70, 72 and 74, 76 respectively. In another application example, not shown in the drawings, traction bands mounted on distal and proximal phalanges may be connected by a single spring module, spanning two interphalangeal joints and the middle phalange.

In the applications or modes of operation shown in Fig. 1 both of the mutually perpendicular orientations of directional axes 50a, 52a and 54a, 56a provided by the single and dual connecting elements 46, 48 are utilized. This orientation of directional axes is usefully described with reference to the longitudinal axis of the digit on which the traction band is mounted. Thus on the first digit or finger 12 the directional axes in use 52a, 56a are perpendicular to (and coplanar with) the longitudinal axis 12a of the finger 12. On the finger 14 most of the directional axes in use 50a, 54a are also perpendicular to the longitudinal axis 14a of the finger but oriented in a plane parallel to but not intercepting the longitudinal axis of the finger or the cylindrical surface formed by the band.

Fig. 1 illustrates three "traction" modes of application of the traction system each using two traction bands 28. These three modes are exemplified by the two traction band pairs (with associated spring modules) spanning the distal interphalangeal joints 70, 72 of the fingers 12 and 14 respectively and a third, extending laterally between the two fingers 12, 14 adjacent their tips. At each pair a spring module 30 has been deflected into a bow configuration and is firmly connected to the connecting elements 48 of the respective pairs of traction bands. It is the nature of the spring 30 to attempt to straighten itself so that each pair of traction bands is subjected to a separating force biasing them apart. The general direction of the separating forces is indicated by arrows 82. Thus the interphalangeal joints (70, 72) of the fingers 12, 14 respectively are subjected to traction by forces (82) approximately parallel to the longitudinal axes 12a 14a of the digits.

The traction system combinations spanning the joints 70, 72 may be installed primarily for traction but there are potentially beneficial secondary force effects. On finger 12 the traction producing springs are dorsally and vertically (radially) oriented so that a secondary effect of the springs tend to extend the finger (that is to straighten a finger which has been bent or flexed downwards). On finger 14 the traction producing springs 30 are also mounted dorsally but extend horizontally (tangentially, in the general plane of the hand). In this orientation the secondary force effect is to flex the finger due to the offset of the spring from the joint. The mode of mounting may be chosen according to the secondary effect that is desired or indicated by the condition of the digit.

The spring 30 connected between the fingers 12, 14 at the finger tip end 24 of the arrangement produces a digit separating force. The "vertical" orientation of the spring 30 leaves the patient more freedom to flex and extend the fingers 12 and 14 relative to one another than would a horizontal orientation which may also be used if a suitable connecting element were provided.

"Vertical" (radial) and "horizontal" (tangential) orientations of spring modules 30 have been discussed, referring to Fig. 1. But note that in Fig. 1 all traction bands are mounted with their connecting elements 46, 48 disposed dorsally (except for the laterally disposed connecting element 46a discussed below). The traction system of the invention may also be used with other dispositions of the connecting element, including, but not limited to, palmar ("inside" the hand) and on the lateral sides of the digits.

A particular application of lateral disposition of connecting element is indicated in Figure 1. Connecting element 46a is where element 46 would be if the band 26 was rotated 90 degrees on the finger 12. In this application only a single traction band is used. The connected spring 30a is bowed and is anchored by bearing against the crotch between the fingers, represented schematically at 20. The spring end may be "cushioned" for comfort, for example with an anchoring sleeve of soft rubber tubing 84. This very compact and simple arrangement may be used for applying a therapeutic force to a metacarpophalangeal joint 78, 80. (Note - for clarity in the drawing the spring 30a is shown extending downwards. Upward extension would usually be more convenient.)

It is a particular advantage of the system according to the invention that the biasing element (spring 30) usually bridges the portion of the extremity being treated (an interphalangeal joint for example) so that there is no direct contact of the biasing element with the treated portion and it may remain exposed. And in all arrangements of the modules of the system there is only limited encumbrance and limited loss of mobility of the hand. The traction bands (26, 28) are only relatively narrow "bandages". Bowed springs (30) do extend away from the digits with some potential for obstruction but the choice of orientation of directional axis of connecting element used and the orientation of traction band itself on the digit may be selected to minimize any obstruction problem in a particular application.

Mounting of a traction band/spring combination on a digit may be facilitated by using a "restraining" tool 32 as indicated in Figures 4 and 5. In the exemplary form shown in Figure 1 one side of the tool 32 provides primarily the function of maintaining the bowed configuration of spring 30 during mounting - see inner end surfaces 86, in Figs. 1 and 4. Once the bands (26, 28) are secured the tool is slipped off the spring 30, leaving it free to exert its axial traction force (82). The other side of the tool 32 provides spaced apart "flats" or wrench surfaces 88 for embracing connecting elements (46, 48) and holding them against rotation - see Figs. 1 and 5 and description below. A closed loop form is shown for the tool but other forms are possible.

The traction system of the invention may also be used to produce lateral opposing forces on the opposite sides of an interphalangeal joint. This is done by storing torsional energy (by means of torsional deflection) in a spring module 30 before mounting the traction bands onto the digit. The procedure is indicated in Fig. 5. With the spring 30 in the position shown, the traction band 26 may be deflected or rotated through 180 degrees or multiples of 180 degrees, to "wind up" the spring 30. The spring may then be realigned to move the connecting element 46 up into engagement with the flats 88 of the tool 32, to prevent the spring from unwinding while the arrangement is being installed on the digit. When the traction bands 26, 28 are secured to the digit the tool 32 may be removed leaving the digit subject to the torque stored in the spring. The general direction of the lateral forces produced on the opposite sides of an interphalangeal joint is indicated by arrows 90 at proximal interphalangeal joint 74 of digit 12 in Fig. 1. Clearly the spring 30 may be wound in either direction to provide lateral forces in a desired direction. The application of such opposing lateral forces at an interphalangeal joint may assist in correcting misalignments brought about by arthritis and other conditions affecting normal movement of the digits, and in relieving associated pain.

A limited number of combinations of the elements of the invention, variations of structure, and modes of application have been described above to indicate the versatility of the system. Other useful variations will be apparent to a person of ordinary skill in the art and such variations are intended to fall within the scope of the claims which follow.

## Claims

1. A band (26, 28) for mounting on a digit (12, 14) of an extremity and facilitating the application of a manipulative force to the digit, the band being engageable with the digit so as to substantially encircle it and so grip the digit that the band is displaced neither axially nor rotationally when the band is subjected to an externally applied manipulative force, the application of the band to the digit establishing a generally cylindrical external surface (36) of the band, the cylindrical surface having a longitudinal axis coinciding approximately with the longitudinal axis (12a, 14a) of the digit, the band being characterized in that the cylindrical surface (36) carries at least one connecting element (46, 48), said element being shaped so as to define at least one directional axis (50a, 52a, 54a, 56a) and to be connectible with an elongated resilient force exerting member (30, 30a), said member having opposite ends (60) and each end having a longitudinal axis (64, 66), and so that, when connected, the at least one directional axis (50a, 52a, 54a, 56a) and the longitudinal axis (64, 66) of the force exerting member end to which it is connected are parallel.

2. The band (26, 28) of claim 1 wherein, in use, the at least one directional axis (50a, 52a, 54a, 56a) is perpendicular to the longitudinal axis (12a, 14a) of the digit.

3. The band (26, 28) of claim 1 or 2 wherein the connecting element (46, 48) includes a hollow generally cylindrical receptacle (50,52, 54, 56) having a longitudinal axis (50a-56a) and said axis defines the at least one directional axis.

4. The band (26, 28) of claim 1 2 or 3 wherein the connecting element (46, 48) is configured to define a second directional axis (50a-56a) perpendicular to the at least one directional axis (50a-56a).

5. The band (26, 28) of claim 1, 2, 3 or 4 further including a second connecting element (46, 48), said element being configured to define at least one directional axis (50a-56a).

6. A system for applying therapeutic forces to an elongated body member (12, 14) at a joint (70-80) of the member, the member having a longitudinal axis (12a, 14a) and the system including a pair of longitudinally spaced anchoring means (28-28, 28-26, 26-84) including a first anchoring means comprising a band (26, 28) for substantially encircling and gripping a first portion of the body member (12, 14), and elongated resilient biasing means (30, 30a) extending between the members of the pair of anchoring means, the system being characterized in that the elongated resilient biasing means (30, 30a) is connected and configured so as to bias apart the members of the pair of anchoring means in a direction generally parallel to the longitudinal axis (12a, 14a) of the body member.

7. The system of claim 6 wherein the second member of the pair of anchoring means (28-28, 28-26) comprises a second band (26, 28) for substantially encircling and gripping a second portion of the body member (12, 14), and wherein said first and second bands (26, 28) are disposed, respectively, on opposite sides of at least one of the joints (70-76) of the body member.

8. The system of claim 6 or 7 wherein the biasing means (30, 30a) comprises an elongated spring having a length greater than the spacing between the members of the pair of anchoring means (28, 26, 84) so that in use the spring forms a bow.

9. The system of claim 8 wherein the spring (30, 30a) is a coil spring wound with initial tension so that it may store energy both by bending and by torsional deflection.

10. The system of claim 7 8, or 9 wherein in use, each band (26, 28) is also secured to its respective body member portion so as to be held against rotational displacement relative to the body member portion and each opposite end of the biasing means (30) is connected to one of the first and second bands (26, 28) and is fixed in relation to the band to which it is connected and wherein relative torsional deflection of the respective opposite ends stores torsional energy in the biasing means so that a torque may be applied to each band (26, 28) by the biasing means (30) and opposing lateral forces generated at the joint (70-76).

11. The traction system of claim 8 9, or 10 wherein the system also includes a tool (32) for embracing the spring (30, 30a) when, in preparation for application to a body member, (12, 14) the spring has been configured into a bow and holding it in said configuration so as to facilitate application of elements of the system to the body member.

12. The traction system of claim 11 wherein the tool (32) includes wrench surfaces (88) for engaging and preventing rotation of the connecting element (46-48) so that torsional energy imparted to the resilient element (30, 30a) by torsionally deflecting an end of said element may be stored in the resilient element prior to installation of the system to the body member (12, 14) and removal of the tool (32) leaves the resilient member (30, 30a) free to apply its torsional energy to the body member.

FIG. 1

FIG. 2

FIG. 3

FIG. 4

FIG. 5

**European Patent Office**

# EUROPEAN SEARCH REPORT

Application Number

| DOCUMENTS CONSIDERED TO BE RELEVANT | | | EP 91304435.0 | |
|---|---|---|---|---|
| Category | Citation of document with indication, where appropriate, of relevant passages | Relevant to claim | CLASSIFICATION OF THE APPLICATION (Int. Cl.5) | |
| D,A | US - A - 4 220 334 (KANAMOTO) * Fig. 1,3; abstract; claims 1,4,6,9,10-12 * | 1,6,7, 8,10 | A 63 B 23/16 | |
| D,A | US - A - 3 533 405 (COLLINS) * Fig.; abstract * | 1,6,7, 8,10, 11 | | |
| A | US - A - 3 189 025 (YAKLIN) * Fig.; claims 1,2 * | 1,2,6, 8 | | |
| A | US - A - 494 197 (HALL) * Fig. 1,9; claims 1-5 * | 1,6,7, 10 | | |
| A | US - A - 248 980 (ATKINS) * Fig.; claims 1,2 * | 1,6,8 | | |
| | | | TECHNICAL FIELDS SEARCHED (Int. Cl.5) | |
| | | | A 63 B 23/00 | |

The present search report has been drawn up for all claims

| Place of search | Date of completion of the search | Examiner |
|---|---|---|
| VIENNA | 18-12-1991 | SCHÖNWÄLDER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)